# EUROPÄISCHE PATENTANMELDUNG

(11) **EP 4 456 161 A1**
(43) Veröffentlichungstag der Anmeldung: **30.10.2024**
(21) Anmeldenummer: 24164169.5
(22) Anmeldetag: 18.03.2024
(51) Int. Cl.: H01M 4/04, H01M 4/139, G01N 27/04

(54) **PROZESSANORDNUNG SOWIE VERFAHREN ZUR FERTIGUNG EINER ELEKTRODE FÜR EINE BATTERIEZELLE**

(30) Priorität: 26.04.2023 DE 102023203853
(71) Anmelder: VOLKSWAGEN AG, 38440 Wolfsburg (DE)
(72) Erfinder: Abel, Tobias, 38114 Braunschweig (DE)

(57) **Zusammenfassung**

Die Erfindung betrifft eine Prozessanordnung zur Fertigung einer Elektrode für eine Batteriezelle, in der eine Ableiter Folie (1) als Endlosbahn kontinuierlich durch Bearbeitungsstationen führbar ist, und zwar unter Bildung einer Elektrodenbahn (E), die an einer abschließenden Schneidstation zur Elektrode abgehängt und/oder zugeschnitten wird. Erfindungsgemäß weist die Prozessanordnung eine Messstation (M) mit zumindest einer Messeinrichtung (4) auf, in der der spezifische Widerstand (ρ) einer Aktivmaterial-Schicht (3) und/oder der Übergangswiderstand (Ω) der Aktivmaterial-Schicht (3) zur Ableiter Folie (1) oder ein damit korrelierender Wert messbar ist.

## Beschreibung

Die Erfindung betrifft eine Prozessanordnung zur Fertigung einer Elektrode für eine Batteriezelle nach dem Oberbegriff des Anspruches 1 sowie ein Verfahren zur Fertigung einer solchen Elektrode nach Anspruch 10.

Die Elektroden einer Lithium-lonen-Batteriezelle sind in einer Großserienfertigung in einem kontinuierlichen Prozess fertigbar, bei dem eine Ableiterfolie als Endlosbahn kontinuierlich durch eine Beschichtungsstation geführt wird, in der die Ableiterfolie ein- oder beidseitig mit Aktivmaterial beschichtet wird. Die so gebildete Elektrodenbahn durchläuft anschließend eine Trockenstation, in der das auf die Ableiterfolie beschichtete Aktivmaterial getrocknet wird. Danach folgt eine Kalandrierstation, in der das auf die Ableiterfolie beschichtete Aktivmaterial bis auf eine vordefinierte Schichtdicke verdichtet wird. Der kontinuierliche Prozess endet an einer Schneidstation, in der die Elektrodenbahn zur Elektrode abgehängt und/oder zugeschnitten wird. Die so hergestellte Elektrode wird anschließend in einen Elektroden-/Separatorstapel integriert, der in eine Batteriezelle verbaubar ist.

Die Größe des Innenwiderstands einer solchen Batteriezelle ist von Bedeutung für die Leistungsfähigkeit der Batteriezelle. Der Batteriezellen-Innenwiderstand wird unter anderem durch den spezifischen Widerstand des Elektroden-Aktivmaterials und durch den Übergangswiderstand der Aktivmaterial-Schicht zur Ableiter Folie mitgeprägt. Zur Messung des spezifischen Widerstands der Aktivmaterial-Schicht sowie des Übergangswiderstands ist ein Analysegerät der Firma HIOKO (siehe Prospekt "electrode resistance measurement system RM2610 der Firma HIOKO) bekannt, mit dem der spezifische Widerstand sowie der Übergangswiderstand bereits fertiggestellter Elektroden stichprobenartig messbar sind. Sofern das Analysegerät einen übermäßig hohen Messwert für den spezifischen Widerstand oder den Übergangswiderstand erfasst, müssen sämtliche bereits gefertigten Elektroden aus der gesamten Beschichtungscharge eines Produktions-Intervalls einem Materialausschuss zugeführt werden und sind diese daher nicht mehr für die weitere Batteriezellen-Fertigung einsetzbar.

Aus der DE 10 2014 006 870 A1 ist ein Verfahren sowie eine Vorrichtung zur Messung einer Beschichtungsstärke auf einem Substrat bekannt. Das Verfahren kann speziell auch zur Messung der Schichtdicke einer Aktivmaterial-Schicht auf einer Ableiterfolie zur Herstellung einer Elektrode eingesetzt werden.

Die Aufgabe der Erfindung besteht darin, eine Prozessanordnung sowie ein Verfahren zur Fertigung einer Elektrode für eine Batteriezelle bereitzustellen, in dem eine Qualitätsprüfung der Elektrode mit im Vergleich zum Stand der Technik weniger Verlustmaterial durchführbar ist.

Die Aufgabe ist durch die Merkmale des Anspruches 1 oder des Anspruches 10 gelöst. Bevorzugte Weiterbildungen der Erfindung sind in den Unteransprüchen offenbart.

Die Erfindung geht von einer Prozessanordnung zur Fertigung einer Elektrode für eine Batteriezelle aus. In dem Fertigungsprozess wird eine Ableiterfolie als Endlosbahn kontinuierlich durch Bearbeitungsstationen geführt, und zwar unter Bildung einer Elektrodenbahn. Die Elektrodenbahn wird in einer abschließenden Schneidstation zur Elektrode abgehängt und/oder zugeschnitten. Gemäß dem kennzeichnenden Teil des Anspruches 1 ist in der Prozessanordnung eine Messstation mit einer Messeinrichtung integriert, in der der spezifische Widerstand einer Aktivmaterial-Schicht und/oder der Übergangswiderstand der Aktivmaterial-Schicht zur Ableiter Folie bzw. ein damit korrelierender Wert gemessen wird.

Der erfindungsgemäße Messvorgang findet im Gegensatz zum Stand der Technik nicht erst an der fertiggestellten Elektrode statt. Vielmehr erfolgt der Messvorgang bereits unmittelbar an der noch nicht der Schneidstation zugeführten Elektrodenbahn, das heißt noch vor Fertigstellung der Elektrode. Auf diese Weise ist eine "in-line"-Qualitätsprüfung des spezifischen Widerstands bzw. des Übergangswiderstands zwischen Folie und Beschichtung bereitgestellt. Werden in der Messstation Qualitätsprobleme in einem Elektrodenbahn-Abschnitt erkannt, so wird dieser Elektrodenbahn-Abschnitt einem Materialausschuss zugeführt, während andere (einwandfrei geprüfte) Elektrodenbahn-Abschnitte derselben Beschichtungscharge den normalen Prozess zur Fertigung der Elektroden durchlaufen. Auf diese Weise kann das Verlustmaterial bei der Elektrodenherstellung im Vergleich zum Stand der Technik in hohem Maße reduziert werden.

In einer technischen Umsetzung kann die Prozessanordnung als Bearbeitungsstation folgendes aufweisen: eine Beschichtungsstation, in der eine Ableiterfolie als Endlosbahn ein- oder beidseitig mit Aktivmaterial beschichtbar ist; eine Trockenstation zum Trocknen des auf die Ableiter Folie beschichtete Aktivmaterial-Schicht; und eine Kalandrierstation in der das auf die Ableiterfolie beschichtete Aktivmaterial bis auf eine vordefinierte Schichtdicke verdichtet wird. Der Kalandrierstation ist mittelbar oder unmittelbar die Schneidstation nachgeschaltet, in der die Elektrodenbahn zur Elektrode abgehängt wird.

Die Messstation zur Messung des spezifischen Widerstands und/oder des Übergangswiderstand kann bevorzugt unmittelbar vor oder nach der Kalandrierstation angeordnet werden. Je nach Bedarf kann daher der spezifische Widerstand sowie der Übergangswiderstands bereits zu einem frühen Prozesszeitpunkt erfasst werden, und zwar bevorzugt schon bevor die Elektrodenbahn die Kalandrierstation durchläuft.

Die in der Prozessanordnung eingebundene Messstation kann eine Auswerteeinheit aufweisen, in der ein von der Messeinrichtung erfasster Widerstands-Messwert mit einem in der Auswerteeinheit hinterlegten Sollwert verglichen wird. Bei einer signifikanten Abweichung des aktuell erfassten Widerstands-Messwerts kann ein aktuell vermessener Elektrodenbahn-Abschnitt aus der regulären Elektrodenfertigung ausgegliedert und einem Materialausschuss zugeführt werden, während die anderen Elektrodenbahn-Abschnitte mit einwandfreiem Widerstands-Messwert für die weitere Batteriezellenfertigung eingesetzt werden.

Die Messeinrichtung kann nach Art eines Analysegeräts der Firma HIOKI (siehe Prospekt "electrode resistance measurement system RM2610 der Firma HIOKO) realisiert sein. In diesem Fall kann die Messeinrichtung zumindest ein Mikro-Elektroden-Array aufweisen, dessen Elektroden während der Messung in zerstörungsfreiem Kontakt mit der Oberfläche der Aktivmaterial-Schicht der Elektrodenbahn sind. Das Mikro-Elektroden-Array kann unterteilt sein in ein Spannungsmess-Array sowie in ein Strommess-Array. Die Spannungsmessung sowie die Strommessung können daher voneinander getrennt durchgeführt werden. Mit Hilfe des Mikro-Elektroden-Arrays kann daher ein Elektrodenbahn-Abschnitt an multiplen Punkten mit einem vordefinierten Stromfluss beaufschlagt werden. Die daraus resultierende Potentialverteilung kann mit Hilfe der Elektroden des Spannungsmess-Arrays an multiplen Messpunkten auf der Oberfläche der Aktivmaterial-Schicht erfasst werden. Beim Messvorgang wird der Strom nicht ausschließlich durch die Ableiterfolie geleitet, sondern auch durch die Aktivmaterial-Schicht, so dass sowohl der Übergangswiderstand als auch der spezifische Widerstand messbar ist.

Dem Mikro-Elektroden-Array kann eine Berechnungseinheit zugeordnet sein. In die Berechnungseinheit werden Daten aus dem gemessenen Stromfluss sowie der gemessenen Potential-Verteilung, der Schichtdicke der Aktivmaterial-Schicht sowie des spezifischen Widerstands der Ableiterfolie eingespeist. In der Berechnungseinheit findet auf der Grundlage dieser Daten eine FEM-Simulation statt, anhand der der spezifische Widerstand sowie der Übergangswiderstands des aktuell vermessenen Elektrodenbahn-Abschnittes ermittelt wird.

In einer konkreten Ausführungsvariante kann die Messstation eine Walzenanordnung mit zumindest einer Messwalze aufweisen. Über die Messwalze verläuft die Elektrodenbahn. Die Messeinrichtung kann in diesem Fall unmittelbar auf dem Außenumfang der Messwalze angeordnet sein, wodurch eine prozesstechnisch einfache "in-line"-Qualitätsprüfung ermöglicht ist.

Im Hinblick auf eine prozesssichere Qualitätsprüfung kann die Messeinrichtung nicht unmittelbar auf einem starren Grundkörper der Messwalze angeordnet sein, sondern vielmehr unter Zwischenlage eines elastisch nachgiebigen Puffermaterials auf dem Grundmaterial der Messwalze angeordnet sein. Das Puffermaterial wirkt nach Art einer Überlastungsschutz-Feder, so dass die Messeinrichtung stets mit einer vordefinierten Andruckkraft, die von der Federkonstante des elastisch nachgiebigen Puffermaterials bestimmt wird, gegen die Aktivmaterial-Schicht der Endlosbahn drückt. Der Messvorgang kann in diesem Fall betriebssicher unabhängig von Toleranzabweichungen in der Schichtdicke der Aktivmaterial-Schicht durchgeführt werden.

Der Außenumfang der Messwalze kann außerhalb der Messeinrichtung mit einer abriebsfesten Beschichtung überzogen sein. Auf diese Weise kann ein Walzenmaterial-Abrieb vermieden werden, der zu einer Verunreinigung der Aktivmaterial-Schicht führen würde.

Zur Steigerung der Messgenauigkeit ist es bevorzugt, wenn die Walzenanordnung zwei Messwalzen aufweist, über die Elektrodenbahn mit ihren beiden Seiten läuft. In diesem Fall kann die eine Seite der Elektrodenbahn in Messkontakt mit der ersten Messwalze gebracht werden, während die andere Seite der Elektrodenbahn in Messkontakt mit der zweiten Messwalze bringbar ist.

Nachfolgend ist ein Ausführungsbeispiel der Erfindung anhand der beigefügten Figuren beschrieben.

Es zeigen:

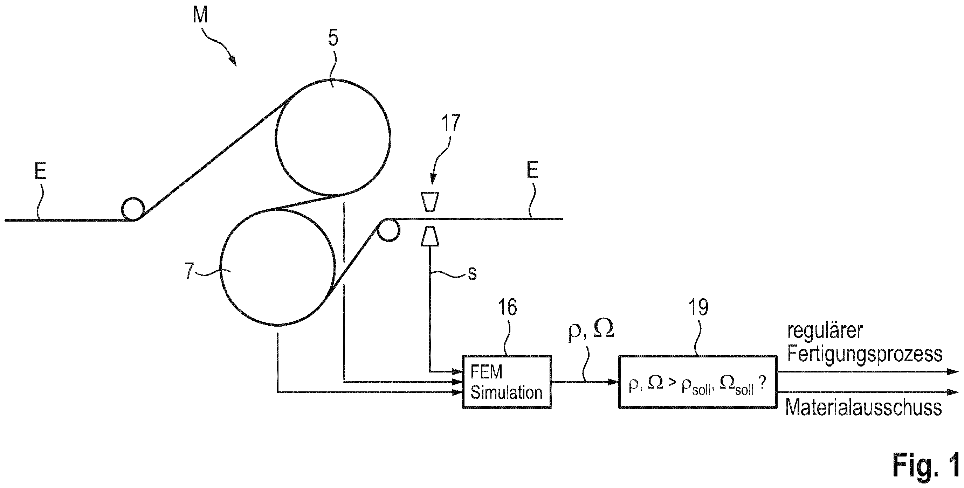

- Figuren 1 bis 5: unterschiedliche Ansichten, anhand derer eine erfindungsgemäße Messstation der Prozessanordnung veranschaulicht sind.
In der Figur 1 ist eine Messstation M zur Qualitätsprüfung gezeigt, die Bestandteil einer nicht näher dargestellten Prozessanordnung ist, in der Elektroden für eine Batteriezelle in einem kontinuierlichen Prozess gefertigt werden. Die Prozessanordnung weist neben der Messstation M weitere nicht gezeigte Bearbeitungsstationen auf, nämlich eine Beschichtungsstation, eine Trockenstation, eine Kalandrierstation sowie eine Schneidstation. In der Beschichtungsstation wird eine Ableiter Folie 1 als Endlosbahn ein- oder beidseitig mit einer Aktivmaterial-Schicht 3 beschichtet, und zwar unter Bildung einer Elektrodenbahn E. Die Elektrodenbahn E wird im weiteren Prozessverlauf durch die Trockenstation geführt, in der die auf die Ableiter Folie 1 beschichtete Aktivmaterial-Schicht 3 getrocknet wird. Im Anschluss daran wird die Elektrodenbahn E durch die Kalandrierstation geführt, in der die auf die Ableiter Folie 1 beschichtete Aktivmaterial-Schicht 3 bis auf eine vordefinierte Schichtdicke verdichtet wird. Danach folgt die Schneidstation, in der die Elektrodenbahn E zur Elektrode abgehängt sowie zugeschnitten wird.

Mit Hilfe einer Messeinrichtung 4 der Messstation M kann der spezifische Widerstand ρ der Aktivmaterial-Schicht 3 sowie der Übergangswiderstand Ω der Aktivmaterial-Schicht 3 zur Ableiter Folie 1 gemessen werden. Der Kern der Erfindung besteht darin, dass die Messstation M in der Prozessanordnung zur kontinuierlichen Elektrodenfertigung integriert ist. Dadurch ist der Messvorgang "in line", also während des kontinuierlichen Fertigungsprozesses unmittelbar an der Elektrodenbahn E durchführbar, das heißt schon zu einem Prozesszeitpunkt vor Fertigstellung der Elektrode.

Wie aus der Figur 1 weiter hervorgeht, weist die Messstation M eine Walzenanordnung aus zwei Messwalzen 5, 7 auf, über die die Elektrodenbahn E mit ihren beiden Seiten läuft. Das heißt, dass gemäß der Figur 1 die eine Seite der Elektrodenbahn E in Messkontakt mit der ersten Messwalze 5 bringbar ist, während die andere Seite der Elektrodenbahn E in Messkontakt mit der zweiten Messwalze 7 bringbar ist. Die Messwalzen 5, 7 weisen die Messeinrichtungen 4 auf, die umfangsverteilt am Außenumfang jeder der Messwalzen 5, 7 positioniert sind. Stromführende Kontakte der Messeinrichtungen 4 können beispielhaft kabelgebunden oder mittels Batterielösung in die jeweilige Messwalze 5, 7 eingebunden sein. Zudem ist den beiden Messwalzen 5, 7 in der Figur 1 eine Präzisions-Dickenmessung 17 nachgeschaltet, mittels der eine lokale Dicke der Elektrodenbahn E erfasst wird.

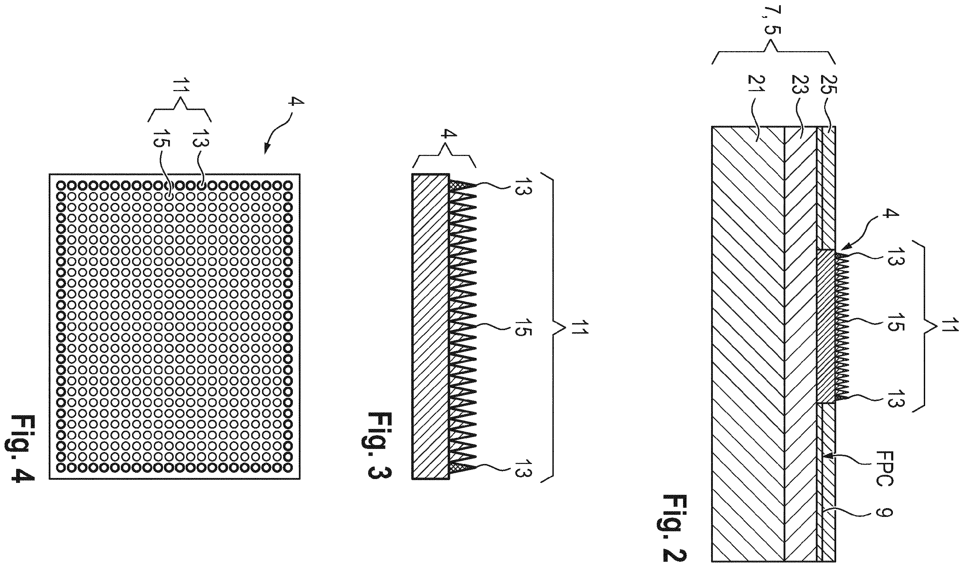

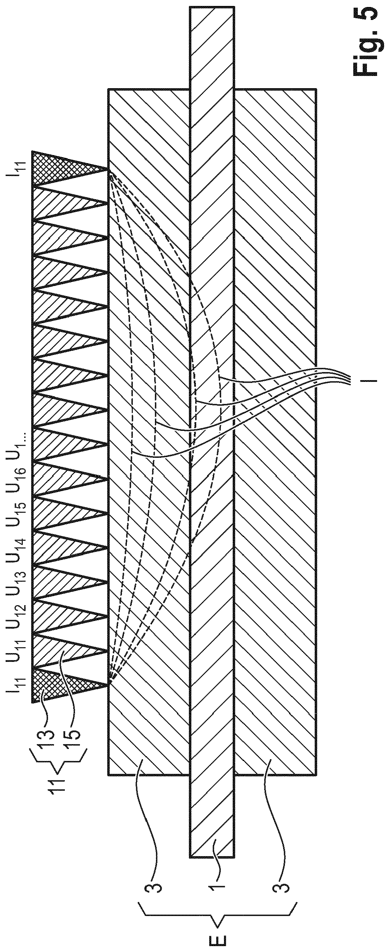

Von den Messeinrichtungen 4 ist in den Figur 2 bis 5 eine Messeinrichtung 4 im Detail dargestellt. Diese ist als ein Chip mit Elektroden-Array 11 realisiert, der in den Figuren 2 bis 4 schematisch insoweit dargestellt ist, als es für das Verständnis der Erfindung erforderlich ist. Die Gesamtbreite des Chips 4 ist in der Größenordnung der Beschichtungsdicke bemessen. Zudem ist der Chip 4 gemäß der Figur 2 Bestandteil eines flexiblen Leiterplatten-Designs (flexible printed circuit) FPC, das aus einem dünnen biegsamen Kunststoffsubstrat 9 besteht, auf dem elektrische Leiterbahnen sowie der Chip 4 aufgebracht sind. Der Chip 4 weist ein Mikro-Elektroden-Array 11 auf, das in ein Strommess-Array 13 sowie in ein Spannungsmess-Array 15 unterteilt ist. Das Strommess-Array 13 besteht aus randseitigen äußeren Strommess-Elektroden, während das Spannungsmess-Array 15 aus inneren Spannungsmess-Elektroden besteht, die von den äußeren Strommess-Elektroden umzogen sind. Die Strommessung sowie die Spannungsmessung erfolgen daher mittels des Strommess-Arrays 13 und des Spannungsmess-Arrays 15 voneinander unabhängig. Während eines Messvorgangs (Figur 5) sind die Elektroden des Mikro-Elektroden-Arrays 11 in zerstörungsfreiem Kontakt mit der Oberfläche der Aktivmaterial-Schicht 3 der Elektrodenbahn E. Ein aktuell zu vermessender Elektrodenbahn-Abschnitt wird mit Hilfe des Mikro-Elektroden-Arrays 11 an multiplen Punkten mit einem vordefinierten Stromfluss I beaufschlagt. Die daraus resultierende Potential-Verteilung wird an multiplen Messpunkten auf der Oberfläche der Aktivmaterial-Schicht 3 von den Elektroden des Spannungsmess-Arrays 15 erfasst.

Die Elektroden des äußeren Strommess-Arrays 13 sind während des Messvorgangs wahlweise als Stromquelle oder -senke nutzbar, während die Elektroden des inneren Spannungsmess-Arrays 15 für eine Spannungsmessung nutzbar sind. Dabei kann jede der Elektroden über einen gesondert ansteuerbaren oder auslesbaren Kanal (dieser kann auch kodiert werden) verfügen. Die Signalverbindung der Elektroden des Chips 4 zur Auswerteeinheit 19 kann durch kabellose (5G, Wifi) oder kabelgebundene, induktive Kontakte nach digitaler Codierung der Signale ausgeführt werden.

Der Messeinrichtung 4 ist in der Figur 1 eine Berechnungseinheit 16 zugeordnet, in die die von dem Strommess-Array 13 und dem Spannungsmess-Array 15 erfassten Daten, die von einer Dickenmessung 17 der Elektrodenbahn E erfassten Daten (das heißt die gemessene Elektrodenbahn-Dicke s) einlesbar sind. Auf der Grundlage dieser Daten sowie des spezifischen Widerstands der Ableiter Folie 1 ermittelt die Berechnungseinheit 16 den spezifischen Widerstand ρ der Aktivmaterial-Schicht 3 sowie den Übergangswiderstand Ω der Aktivmaterial-Schicht 3 zur Ableiter Folie 1 des aktuell vermessenen Elektrodenbahn-Abschnittes. Die Berechnungseinheit 16 arbeitet mittels einer lokalen FEM-Simulation. Von daher erfolgt in der Berechnungseinheit 16 eine Simulation der unter dem Chip 4 befindlichen Aktivmaterial-Schichten 3 mit Hilfe eines Simulationsmodells und der Schichtdickenmessung.

Die ermittelten Widerstands-Werte ρ, Ω werden einer Auswerteeinheit 19 zugeführt, die die Widerstands-Werte ρ, Ω mit entsprechenden Sollwerten ρₛₒₗₗ, Ωₛₒₗₗ vergleicht. Bei einer signifikanten Abweichung der aktuell erfassten Widerstands-Messwerte wird der aktuell vermessene Elektrodenbahn-Abschnitt nicht für die weitere Elektrodenfertigung verwendet, sondern vielmehr einem Materialausschuss zugeführt. Sofern sich die aktuellen Widerstands-Messwerte ρ, Ω ohne größere Abweichung im Bereich der Sollwerte ρₛₒₗₗ, Ωₛₒₗₗ befinden, wird der aktuell vermessene Elektrodenbahn-Abschnitt für die weitere Batteriezellenfertigung verwendet.

In der Figur 5 ist mit gestrichelter Linie ein Stromfluss I zwischen Strommess-Elektroden des Strommess-Arrays 13 angedeutet. Demnach fließt der Strom I nicht ausschließlich durch die Ableiterfolie 1, sondern auch durch die Aktivmaterial-Schicht 3, so dass sowohl der spezifische Widerstand ρ der Aktivmaterial-Schicht 3 als auch der Übergangswiderstand Ω der Aktivmaterial-Schicht 3 zur Ableiter Folie 1 mit großer Genauigkeit messbar ist.

In der Figur 2 ist grob schematisch der Materialaufbau der Messwalzen 5, 7 gezeigt. Demnach weist die jeweilige Messwalze 5, 7 ein starres Grundmaterial 21 auf. Das flexible Leiterplatten-Design FPC ist nicht unmittelbar, sondern vielmehr unter Zwischenlage eines elastisch nachgiebigen Puffermaterials 23 auf dem starren Grundmaterial 21 der Messwalze 5 angeordnet. Das Puffermaterial 23 wirkt nach Art einer Überlastschutz-Feder, mittels der die Messeinrichtung 4 (das heißt der Chip) stets mit einer vordefinierten Andruckkraft gegen die Aktivmaterial-Schicht 3 der Endlosbahn E drückt, und zwar unabhängig von zum Beispiel Toleranzabweichungen in der Schichtdicke s der Elektrodenbahn E. Zudem ist die Messwalze 5 am Außenumfang außerhalb der Messeinrichtung 4 mit einer abriebsfesten Beschichtung 25 (aus zum Beispiel PTFE) überzogen, wodurch ein betriebsbedingter Walzenmaterial-Abrieb vermieden wird, der zu einer Verunreinigung der Aktivmaterial-Schicht 3 führen könnte.

Alternativ zum gezeigten Ausführungsbeispiel kann der Chip 4 auch zum Beispiel direkt auf die Messwalze 5. 7 aufgebracht oder in eine feste Schicht eingebettet sein. Der Walzenaufbau kann ferner auch ohne abriebsfeste Schicht 25 realisiert werden. Zudem kann die abriebsfeste Schicht 25 - anstelle von PTFE - auch aus Metallen, etwa Chrom, Nickel, oder aus Keramikbeschichtungen realisiert werden. Zudem ist ein weiteres Ausführungsbeispiel ein Chip mit gefederten, einzelnen Kontakten. Dies kann durch eine beliebige Art Feder gewährleistet werden. Wichtig ist, dass es im Betrieb keinen elektrischen Kontakt zwischen den verschiedenen Elektroden gibt.

### Bezugszeichenliste

- 1: Ableiterfolie
- 3: Aktivmaterial-Schicht
- 4: Messeinrichtung
- 5, 7: Messwalzen
- 9: Kunststoffsubstrat
- 11: Mikro-Elektroden-Array
- 13: Strommess-Array
- 15: Spannungsmess-Array
- 16: Berechnungseinheit
- 17: Dickenmessung
- 19: Auswerteeinheit
- 21: Walzen-Grundmaterial
- 23: Puffermaterial
- 25: abriebsfeste Beschichtung
- E: Elektrodenbahn
- M: Messstation
- FPC: flexibles Leiterplatten-Design
- ρ: spezifischer Widerstand
- Ω: Übergangswiderstand
- s: Schichtdicke der Elektrodenbahn E
- I: Stromfluss

## Patentansprüche

1. Prozessanordnung zur Fertigung einer Elektrode für eine Batteriezelle, in der eine Ableiterfolie (1) als Endlosbahn kontinuierlich durch Bearbeitungsstationen führbar ist, und zwar unter Bildung einer Elektrodenbahn (E), die an einer abschließenden Schneidstation zur Elektrode abgelängt und/oder zugeschnitten wird, **dadurch gekennzeichnet, dass** die Prozessanordnung eine Messstation (M) mit zumindest einer Messeinrichtung (4) aufweist, in der der spezifische Widerstand (p) einer Aktivmaterial-Schicht (3) und/oder der Übergangswiderstand (Ω) der Aktivmaterial-Schicht (3) zur Ableiter Folie (1) oder ein damit korrelierender Wert messbar ist, und dass insbesondere der Messvorgang unmittelbar an der Elektrodenbahn (E) (in line), das heißt noch vor der Fertigstellung der Elektrode durchführbar ist.

2. Prozessanordnung nach Anspruch 1, **dadurch gekennzeichnet, dass** die Prozessanordnung als Bearbeitungsstationen aufweist:
- eine Beschichtungsstation, in der die Ableiter Folie (1) ein- oder beidseitig mit der Aktivmaterial-Schicht (3) beschichtbar ist,
- eine Trockenstation zum Trocknen der auf der Ableiter Folie (1) beschichteten Aktivmaterial-Schicht (3),
- eine Kalandrierstation, in der die auf die Ableiter Folie (1) beschichtete Aktivmaterial-Schicht (3) bis auf eine vordefinierte Schichtdicke verdichtbar ist, und dass die Schneidstation der Kalandrierstation prozesstechnisch nachgeschaltet ist.

3. Prozessanordnung nach Anspruch 1 oder 2, **dadurch gekennzeichnet, dass** die Messstation (M) der Kalandrierstation prozesstechnisch unmittelbar vor- und/oder nachgeschaltet ist, so dass der spezifische Widerstand (p) bzw. der Übergangswiderstand (Ω) vor und/oder nach dem Kalandriervorgang messbar ist.

4. Prozessanordnung nach Anspruch 1, 2 oder 3, **dadurch gekennzeichnet, dass** die Messstation (M) eine Auswerteeinheit (19) aufweist, die einen von der Messeinrichtung (4) erfassten Widerstands-Messwert (ρ, Ω) mit einem Sollwert (ρₛₒₗₗ, Ωₛₒₗₗ) vergleicht, und dass insbesondere bei einer signifikanten Abweichung des aktuell erfassten Widerstands-Messwerts (ρ, Ω) der aktuell vermessene Elektrodenbahn-Abschnitt nicht für die Elektrodenfertigung verwendet wird, sondern einem Materialausschuss zugeführt wird.

5. Prozessanordnung nach einem der vorhergehenden Ansprüche, **dadurch gekennzeichnet, dass** die Messeinrichtung (4) zumindest ein Mikro-Elektroden-Array (11) aufweist, dessen Elektroden während der Messung in zerstörungsfreiem Kontakt mit der Oberfläche der Aktivmaterial-Schicht (3) sind.

6. Prozessanordnung nach Anspruch 5, **dadurch gekennzeichnet, dass** mittels des Mikro-Elektroden-Arrays (11) ein Elektrodenbahn-Abschnitt an multiplen Punkten mit einem vordefinierten Stromfluss (I) beaufschlagbar ist, und dass die daraus resultierende Potential-Verteilung an multiplen Messpunkten auf der Oberfläche der Aktivmaterial-Schicht (3) erfassbar ist, und/oder dass das Mikro-Elektroden-Array (11) unterteilt ist in ein Strommess-Array (13), dessen Elektroden als Stromquelle sowie Stromsenke zur Erzeugung des Stromflusses (I) wirken, und in ein Spannungsmess-Array (15), dessen Elektroden die aus dem Stromfluss (I) resultierende Potential-Verteilung messen, und/oder dass insbesondere dem Mikro-Elektroden-Array (11) eine Berechnungseinheit (16) zugeordnet ist, die auf der Grundlage des Stromflusses (I), der erfassten Potential-Verteilung, der Schichtdicke (s) der Aktivmaterial-Schicht (3) und des spezifischen Widerstands der Ableiterfolie (1) den spezifischen Widerstand (p) der Aktivmaterial-Schicht (3) sowie den Übergangswiderstand (Ω) berechnet, und zwar insbesondere mittels einer FEM-Simulation.

7. Prozessanordnung nach einem der vorhergehenden Ansprüche, **dadurch gekennzeichnet, dass** die Messstation (M) eine Walzenanordnung mit zumindest einer Messwalze (5, 7) aufweist, über die die Elektrodenbahn (E) läuft, und dass insbesondere die Messeinrichtung (4) am Außenumfang der Messwalze (5, 7) angeordnet ist, oder dass die Messstation (M) einen mit der Elektrodenbahn (E) bewegungsgekoppelten linear verstellbaren Schlitten aufweist, mittels dem die Messeinrichtung (4) über eine lineare Messstrecke in Messkontakt mit der Elektrodenbahn (E) bringbar ist.

8. Prozessanordnung nach Anspruch 7, **dadurch gekennzeichnet, dass** die Messeinrichtung (4) nicht unmittelbar auf einem starren Grundmaterial (21) der Messwalze (5, 7) angeordnet ist, sondern unter Zwischenlage eines elastisch nachgiebigen Puffermaterials (23) auf dem Grundmaterial (21) der Messwalze (5, 7) angeordnet ist, so dass insbesondere die Messeinrichtung (4) mit einer vordefinierten Andruckkraft gegen die Aktivmaterial-Schicht (3) der Elektrodenbahn (E) drückt, und zwar bevorzugt unabhängig von Toleranzabweichungen in der Schichtdicke der Aktivmaterial-Schicht (3), und dass insbesondere der Außenumfang der Messwalze (5, 7) im Bereich außerhalb der Messeinrichtung (4) mit einer abriebsfesten Beschichtung (25) überzogen ist, um eine Verunreinigung der Aktivmaterial-Schicht (3) durch Walzenmaterial-Abrieb zu vermeiden, und/oder dass die Walzenanordnung zwei Messwalzen (5, 7) aufweist, über die die Elektrodenbahn (E) mit ihren beiden Seiten läuft, so dass die eine Seite der Elektrodenbahn (E) in Messkontakt mit der ersten Messwalze (5) bringbar ist, und die andere Seite der Elektrodenbahn (E) in Messkontakt mit der zweiten Messwalze (7) bringbar ist.

9. Verfahren zur Fertigung einer Elektrode in einer Prozessanordnung nach einem der vorhergehenden Ansprüche.
